# EUROPÄISCHE PATENTANMELDUNG

(11) **EP 1 002 870 A1**
(43) Veröffentlichungstag der Anmeldung: **24.05.2000**
(21) Anmeldenummer: 99250391.2
(22) Anmeldetag: 04.11.1999
(51) Int. Cl.: C12N 15/85, C12N 15/11, C12Q 1/02, C12Q 1/68, G01N 33/50

(54) **Androgen- und Progesteron-Rezeptor bindendes Hormon Response Element**

(30) Priorität: 20.11.1998 DE 19855013
(71) Anmelder: Schering Aktiengesellschaft, 13353 Berlin (DE)
(72) Erfinder: Haendler, Bernhard, 13465 Berlin (DE)

(57) **Zusammenfassung**

Es wird ein neues Hormon Response Element, das den Androgen- und den Progesteronrezeptor bindet, und seine Verwendung beschrieben.

## Beschreibung

Die Erfindung betrifft ein neues HRE (Hormon Response Element, Hormonrezeptorbindungstelle), das durch den Androgen- und den Progesteronrezeptor gebunden wird, seine Sequenz und seine Verwendung.

Steroidrezeptoren sind Transkriptionsfaktoren, die eine Ligandenbindungstelle, eine DNA-Bindungstelle und mehrere Transaktivierungsfunktionen besitzen. Wenn der Ligand, das Steroid, bindet, ändert sich die Konformation des Rezeptors. Durch diese Konformationsänderung kann der Rezeptor ein Dimer bilden und an eine spezifische doppelsträngige DNA Sequenz, das sogenannte HRE (Hormon Response Element, Hormonrezeptorbindungstelle) binden und mit Koaktivatoren und anderen Transkriptionsfaktoren interagieren. Als Folge davon wird die Transkription des Zielgens, also des Gens, das durch das HRE reguliert wird, aktiviert. Diese HREs sind gewöhnlich Bestandteile von Promotorregionen solcher Zielgene, können sich aber auch in den Intronbereichen oder im 3' Teil des Gens befinden. Für das HRE des Androgenrezeptors ist eine Konsensus-Sequenz GG(T/A)ACAnnnTGTTCT ermittelt worden (Roche et al. 1992, Mol. Endocrinol. 6, 2229-2235). n bezeichnet dabei jedes beliebige Nukleotid. Die Halbelemente, das sind die definierten Nukleotide vor bzw. nach der "nnn" Sequenz, sind annähernd palindromisch. Es wurde gezeigt, daß die Sequenz GGTACAtctTGTTCA, die im Promotor des stark Androgen-regulierten CRISP-1-Gens vorkommt, eine hohe Bindungsaffinität für den Androgenrezeptor aufweist (Schwidetzky et al. 1997, Biochem. J. 321, 325-332). Diese Sequenz wird im folgenden Konsensus-HRE bezeichnet. Sie wird jedoch nicht nur vom Androgenrezeptor sondern auch vom Glucokortikoid- und Progesteronrezeptor erkannt. Dagegen bindet das HRE der Promotorregion des Probasin-Gens selektiv den Androgenrezeptor und nicht den Glucokortikoidrezeptor (F. Claessens et al. 1996, J. Biol. Chem. 271, 19013-19016). Über die Bindung des Progesteronrezeptors liegen hier keine Angaben vor. Durch eine *in vitro* Selektionsmethode wurde kürzlich eine neue DNA-Sequenz gefunden, genannt IDR17, die den Androgenrezeptor binden kann (Zhou et al. 1997, J. Biol. Chem. 272, 8227-8235). Verschiedene Versuche zeigten, daß diese Sequenz eine hohe Selektivität für den Androgenrezeptor hat im Vergleich zum Glucokortikoidrezeptor. Ein Vergleich mit dem Progesteronrezeptor wurde nicht beschrieben. Die Sequenz IDR17 ist eine *in vitro* gefundene Sequenz; es gibt keine Hinweise, ob sie auch *in vivo* vorkommt und dort eine Funktion als HRE hat.

Die Kenntnis über HREs ist hilfreich bei der Entwicklung von Medikamenten für die Behandlung von Hormon-abhängigen Krankheiten. Bei der Behandlung von Prostatakrebs mit Antiandrogenen, die die Bindung des Androgenrezeptors an das Konsensus-HRE hemmen, zeigt sich häufig, daß nicht alle Androgen-regulierten Gene herrunterreguliert werden und/oder die Antiandrogene nach einer gewissen Behandlungszeit nicht mehr wirksam sind. Diese Befunde weisen daraufhin, daß es unterschiedliche HREs geben muß, die verschiedene Gene regulieren und für die Hormon-Abhängigkeit verantwortlich sind. Für eine erfolgreiche Hormon-Therapie ist es deshalb wünschenswert, weitere HREs, an die der Androgenrezeptor bindet, zu kennen. Es könnten dann spezifisch wirksame natürliche Hormone, synthetische Hormone oder Antihormone identifiziert werden. Diese können dann die für die Krankheiten relevanten Gene an- oder abschalten. Bisher sind nur einige Gene, die durch Androgene reguliert werden, und ihre HREs bekannt. Beispiele hierfür sind das Probasin-Gen und das C3(1) Gen, die beide in Ratten-Prostata durch Androgen induziert werden (F. Claessens et al. 1989, Biochem. Biophys. Res. Commun. 164, 833-840; P.S. Rennie et al. 1993, Mol. Endocrinol. 7, 23-36). In Ratten-Nebenhoden wird das Pem-Gen Androgen-abhängig exprimiert (S. Maiti et al. 1996, J. Biol. Chem. 271, 17536-17546). Die ungefähre Lage von zwei Transkriptionsinitiationsstellen wird beschrieben, das HRE ist allerdings nicht bekannt.

Wünschenswert ist es, ein neues HRE für den Androgenrezeptor zur Verfügung zu haben. Die Erfindung liefert ein neues HRE, das den Androgen- und den Progesteronrezeptor bindet und das
a. eine DNA-Sequenz der allgemeinen Formel (N)ₓTCTCATTCTGTTCC, wobei N unabhängig voneinander und in beliebiger Kombination A, T, C oder G ist und x gleich 0-3 ist,
b. eine Variation der unter a. genannten Sequenz oder
c. eine DNA-Sequenz, die komplementär zu der unter a. oder b. genannten DNA-Sequenz ist, umfaßt.

Eine Variation der Sequenz kann z.B. eine allelische Variation sein. Mit allelischer Variation ist eine Mutation gemeint, d.h. eine Änderung der DNA-Sequenz, die durch natürliche Deletion, Addition oder Substitution von Nukleotiden gekennzeichnet ist. Jede dieser Änderungen kann allein oder in Kombination mit anderen Änderungen, einmal oder mehrfach in der DNA-Sequenz auftreten. Durch diese Änderungen ändern sich die Bindungseigenschaften des HRE nur unwesentlich. Allelische Varianten sollen eine mindestens 80 %ige, bevorzugt eine 85 %ige oder am meisten bevorzugt 90 %ige Sequenzidentität zu der unter a. genannten Sequenz aufweisen. *In vivo* liegt die erfindungsgemäße DNA-Sequenz als Doppelstrang vor, d.h. sie bindet an die ihr komplementäre DNA-Sequenz.

Außerdem kann eine Variation eine Sequenz sein, die unter stringenten Bedingungen mit der erfindungsgemäßen Sequenz hybridisiert. Stringente Bedingungen sind für ein HRE mit 17 Nukleotiden eine Hybridisierungstemperatur von 35°C, bevorzugt 38°C, am meisten bevorzugt 41°C in einem QuickHyb-Puffer (Stratagene).

An das erfindungsgemäße HRE bindet spezifisch der Androgen- und der Progesteronrezeptor dagegen nicht der Glucokortikoidrezeptor. Das erfindungsgemäße HRE reagiert sehr viel empfindlicher auf den Androgen- und den Progesteronrezeptor als das Konsensus-HRE und das IDR17-HRE, was zu einer stärkeren Induktion des Zielgens führt (s. Beispiel 4). Kotransfektion mit Expressionskonstrukten, die für Proteine kodieren, von denen anzunehmen ist, daß sie mit dem Androgenrezeptor in Wechselwirkung treten, führt zu einer stärkeren Stimulierung des erfindungsgemäßen HREs (ARE17) im Vergleich zu dem Konsensus-HRE (s. Beispiel 4). Die Androgenstimulation des erfindungsgemäßen HRE läßt sich schon durch 125 ng/ml Geldanamycin hemmen im Gegensatz zum Konsensus-HRE, das dadurch eher stimuliert wird und erst bei höherer Geldanamycin Konzentration gehemmt wird (s. Beispiel 4). Geldanamycin ist eine Substanz, die die Aktion von Steroidrezeptoren hemmt (D. F. Smith et al. 1995, Cell. Biol. 15, 6804-6812). HREs, die von dem Östrogen-, Glucocorticoid- oder Mineralocorticoidrezeptor gebunden werden, bestehen aus 2 fast palindromischen Halbelementen (Evans et al. US5,597,693). Auch das Konsensus-HRE für den Androgenrezeptor hat eine fast palindromische Struktur. Das erfindungsgemäße HRE hat dagegen keine palindromische Struktur und kann eher als "direct repeat" des Halbelements TGTTCT gesehen werden. Dieser strukturelle Unterschied und die oben genannten Unterschiede deuten darauf hin, daß die Interaktion zwischen dem Androgenrezeptor und dem erfindungsgemäßen HRE anders ist als zwischen dem Androgenrezeptor und dem Konsensus-HRE. Deshalb sollte die Interaktion mit dem erfindungsgemäßen HRE durch einen anderen Mechanismus und durch andere Androgene bzw. Antiandrogene modifiziert werden können als die Interaktion mit dem Konsensus-HRE.

Bevorzugt ist ein erfindungsgemäßes HRE, bei dem x = 0 ist und die DNA-Sequenz Seq. ID No.3 entspricht. Weiterhin bevorzugt ist ein erfindungsgemäßes HRE, bei dem x=3 ist. Am meisten bevorzugt ist ein erfindungsgemäßes HRE, bei dem x=3 ist und N=AGA und die DNA-Sequenz Seq. ID No. 1 entspricht.

Die erfindungsgemäße HRE-DNA-Sequenz kann zur Detektion von Genen, die durch Androgen und/oder Progesteron reguliert werden, verwendet werden. Dies kann in menschlichem oder tierischem Gewebe geschehen. Dadurch können neue Gene identifiziert werden oder die Androgen- und/oder Progesteron-Regulation bekannter Gene sowie unbekannter Gene detektiert werden. Die Detektion kann z.B. durch Computer-gestützte Analyse von DNA Datenbanken, DNA-Sequenzierung, oder durch ein Punktmutation-Screeningverfahren (z.B. Sequenzierung von PCR-amplifizierten Genomfragmenten oder single strand conformation polymorphism SSCP) (A.C. Goodeve 1998, Clin. Lab. Haematol. 20, 3-19) erfolgen. Dabei wird die jeweils analysierte DNA-Sequenz mit der erfindungsgemäßen HRE-DNA-Sequenz verglichen. Außerdem kann ein Oligonukleotid mit der erfindungsgemäßen HRE-Sequenz synthetisiert werden. Die Synthese erfolgt dabei nach Routinemethoden wie sie z.B. in M.H. Caruthers et al. 1983, Gene Amplif. Anal. 3, 1-26, beschrieben sind. Das Oligonukleotid kann radioaktiv markiert sein oder einen detektierbaren Rest enthalten wie z.B. Digoxigenin, alkalische Phosphatase, Biotin, Fluorescein, Rhodamine, Texas Red, Oregon Green oder Bodipy Dyes. Mit Hilfe dieses der erfindungsgemäßen HRE-DNA-Sequenz entsprechenden Oligonukleotids können durch Screening von genomischen Banken, einen Southern blot oder *in situ* Hybridisierung oder durch anderen dem Fachmann bekannten Methoden die Gene detektiert werden, die durch das erfindungsgemäße HRE reguliert werden.

Eine erfindungsgemäße HRE Sequenz oder eine durch eine erfindungsgemäße HRE Sequenz regulierte Sequenz kann zur Detektion einer Veränderung der Expression von Androgen oder/und Progesteron regulierten Genen verwendet werden. So können z.B. erkrankte Zellen mit normalen Zellen verglichen werden. Eine durch eine erfindungsgemäße HRE Sequenz regulierte Sequenz kann z. B. eine Sequenz aus dem kodierenden Bereich eines Zielgens sein.

Die Erfindung betrifft weiterhin die Verwendung eines Oligonukleotids entsprechend einer erfindungsgemäßen DNA-Sequenz zur Detektion von Mutationen in Expressions-Regulationselementen, insbesondere HRE's, die durch Androgen oder Progesteron reguliert werden, in menschlichem oder tierischem Gewebe. Dabei kann es sich um normale Zellen, d.h. nicht veränderte Zellen, etablierte Zellinien und um Tumorzellen handeln. Die Detektion solcher Mutationen kann z.B. durch Sequenzierung der aus diesen Geweben oder Zellen isolierten genomischen DNA oder durch ein Punktmutation-Screeningverfahren (A.C. Goodeve 1998, Clin. Lab. Haematol. 20, 3-19) erfolgen. Solche Mutationen können eine Rolle in dem oft beobachteten Relaps bei der Behandlung von Tumorpatienten mit Antihormonen spielen. Die Interaktion des Androgen- oder Progesteronrezeptors nach Bindung von Antagonisten oder Partialagonisten mit dem mutierten HRE kann verändert sein und dadurch zu einer veränderten Expression oder Repression von Genen führen. Die Detektion solcher Mutationen sollte es erlauben, eine Therapie dem Patienten, in dem man solche Mutationen gefunden hat, besser anzupassen.

Die Erfindung betrifft ferner Vektoren umfassend ein erfindungsgemäßes HRE, in operativer Verknüpfung mit dem zu exprimierenden Gen. Dabei kann das erfindungsgemäße HRE in mehreren Kopien vorliegen, vorzugsweise in 1-4 Kopien. Als operative Verknüpfung kann eine minimale Promotersequenz verwendet werden. In der Literatur sind eine Anzahl von Vektoren und minimalen Promotoren bekannt, die verwendet werden können. Es können z.B. die Vektoren pGL3-Basic oder pGL3-Enhancer benutzt werden. Als Promotor kann der TK minimal Promotor (G. R. Reyes et al. 1982, J. Gen.Virol. 62, 191-206) oder vergleichbare minimale Promotoren verwendet werden. Ein minimaler Promotor ist ein Promotor, der nur die TATA-Box und eine Transkriptionsinitiationsstelle hat. Als TATA-Box wird die DNA-Sequenz die durch den Proteinkomplex TFIID erkannt wird, bezeichnet.

Mit dem erfindungsgemäßen Vektor oder einer DNA, die ein erfindungsgemäßes HRE enthält, können Zellen transfiziert werden. Diese transfizierten Zellen sind ebenfalls Gegenstand der vorliegenden Erfindung. Bei den Zellen kann es sich um eukaryotische Zellen, also auch z. B. Hefen handeln. Geeignete Zellen sind z. B. PC-3-Zellen (M. E. Kaighn et al., 1979, Invest. Urol. 17 16-23), LNCaP-Zellen (J. S. Horoszewicz et al., 1983, Cancer Res. 43 1809-1818), CV-1-Zellen (F. C. Jensen et al., 1964, Proc. Natl. Acad. Sci. USA 52, 53-59 ), HeLa-Zellen (T. T. Puck et al., 1956, J. Exp. Med. 103 273-284) und Dunning-Zellen (D. D. Mickey et al., 1977, Cancer Res. 37 4049-4058). Die Transfektion kann durch gängige Methoden, wie z.B. mit den Transfektionsreagenzen Calciumchlorid, FuGene, LipoTAXI, DOTAP, Lipofectin, Lipofectamine, Superfect, oder durch Elektroporation oder mit Hilfe der Gene Gun durchgeführt werden. Methoden für Transfektionen sind in Molecular Cloning, A Laboratory Manual (Sambrook et al., 1989, Cold Spring Harbor Laboratory Press) beschrieben.

Die erfindungsgemäßen Zellen können zur Expression des mit der HRE-Sequenz operativ verknüpften Gens verwendet werden. Bevorzugt enthält die Zelle ein mit den HRE-Element bindefähiges Polypeptid, welches eine zusätzliche Ligandenbindedomäne aufweist. Besonders bevorzugt ist als Polypeptid der Androgenrezeptor oder der Progesteronrezeptor. Die Zellen können stabil oder transient mit dem Androgenrezeptor- oder Progesteronrezeptor-Gen oder mit Teilen davon oder mit Teilen davon in Kombination mit Transaktivierungsdomäne von anderen Faktoren transfiziert werden und die Expression des Proteins durch Zugabe von einem Androgen oder einem Progestin zu den kultivierten Zellen induziert werden. Teile des Androgen- oder Progesteronrezeptors können z.B. sein die Liganden-bindende Domäne, die Transaktivierungsdomäne und die DNA-bindende Domäne. Transaktivierungsdomänen von anderen Faktoren können z.B. sein Gal 4-Transaktivierungsdomäne oder VP16-Transaktivierungsdomäne.

Die Erfindung betrifft ferner ein Testsystem für hormonrezeptorbindungsfähige Substanzen umfassend
a. eine erfindungsgemäße DNA-Sequenz in operativen Verknüpfung mit einem zu exprimierenden oder zu transkribrierenden heterologen Gen
b. ein Polypeptid, das mit der unter a. beschriebenen erfindungsgemäßen DNA bindefähig ist.

Ein zu exprimierendes Gen kann ein nachweisbares Protein kodieren. Ein zu transkribierendendes Gen kann in eine nachweisbare RNA transkribiert werden.

Weiterhin betrifft die Erfindung ein Testsystem zur Detektion von Testsubstanzen mit androgener Aktivität, wobei
a. in den erfindungsgemäßen Zellen ein Reportergen exprimiert wird und
b. diese Zellen, wenn sie keinen oder nur wenig Androgenrezeptor enthalten, zusätzlich mit einem Expressionsvektor für den Androgenrezeptor transfiziert werden,
c. die Zellen in Gegenwart oder Abwesenheit der Testsubstanzen kultiviert werden und
d. die Veränderung der Expression des Reportergens gemessen wird.

Ferner betrifft die Erfindung ein Testsystem zur Detektion von Testsubstanzen mit antiandrogener Aktivität, wobei
a. in den erfindungsgemäßen Zellen ein Reportergen exprimiert wird und
b. diese Zellen, wenn sie keinen oder nur wenig Androgenrezeptor enthalten, zusätzlich mit einem Expressionsvektor für den Androgenrezeptor transfiziert werden,
c. die Zellen in Gegenwart oder Abwesenheit der Testsubstanzen bei gleichzeitiger Anwesenheit eines Androgens kultiviert werden und
d. die Veränderung der Expression des Reportergens gemessen wird
und ein Testsystem zur Detektion von Testsubstanzen mit Progestin- Aktivität, wobei
a. in den erfindungsgemäßen Zellen ein Reportergen exprimiert wird und
b. diese Zellen, wenn sie keinen oder nur wenig Progesteronrezeptor enthalten, zusätzlich mit einem Expressionsvektor für den Progesteronrezeptor transfiziert werden,
c. die Zellen in Gegenwart oder Abwesenheit der Testsubstanzen kultiviert werden und
d. die Veränderung der Expression des Reportergens gemessen wird.

Schließlich liefert die Erfindung ein Testsystem zur Detektion von Testsubstanzen mit anti-Progestin Aktivität, wobei
a. in den erfindungsgemäßen Zellen ein Reportergen exprimiert wird und
b. diese Zellen, wenn sie keinen oder nur wenig Progesteronrezeptor enthalten, zusätzlich mit einem Expressionsvektor für den Progesteronrezeptor transfiziert werden,
c. die Zellen in Gegenwart oder Abwesenheit der Testsubstanzen bei gleichzeitiger Anwesenheit eines Progestins kultiviert werden und
d. die Veränderung der Expression des Reportergens gemessen wird.

Reportergene können z.B. sein Luciferase-Gen, Chloramphenicolacetyltransferasegen, Urokinasegen, Green Fluorescence Protein-Gen und β-Galaktosidasegen. Ein Androgen kann ausgewählt sein aus der Gruppe bestehend aus R1881, Testosteron, Dihydrotestosteron und Testosteron-Derivaten, und ein Progestin kann ausgewählt sein aus der Gruppe bestehend aus Levonogestrel, Progesteron, R5020, Drospirinon und Dienogest. Eine Substanz mit antiandrogener Aktivität verhindert die Wirkung des Androgens auf den Androgenrezeptor und ein Anti-Progestin hemmt den Effekt von Progesteron oder Progestinen auf den Progesteronrezeptor.

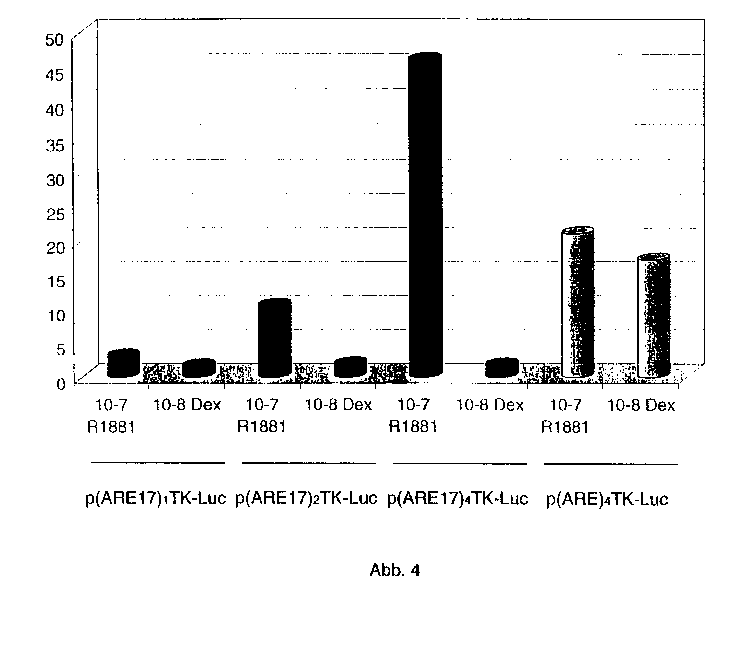

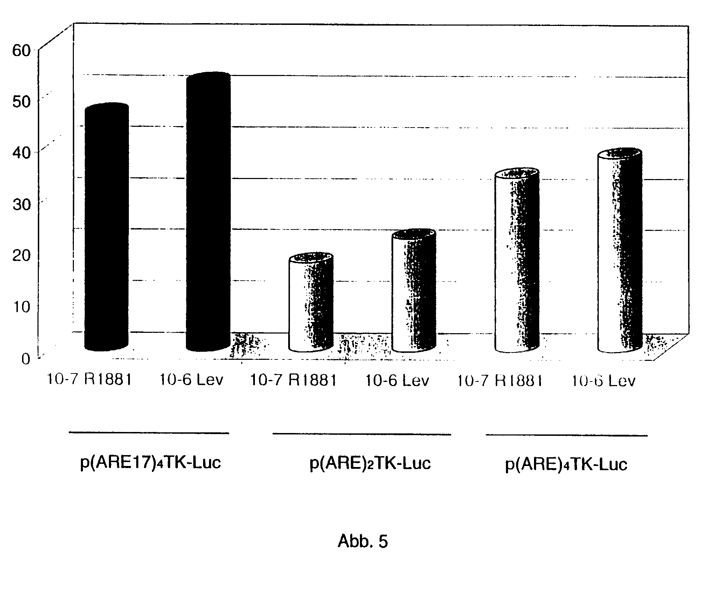

Bevorzugt ist die Verwendung des Luciferase Reporter-Gens und von PC-3-Zellen, die zuvor mit einem Expressionsvektor für den Androgen-Rezeptor stabil (PC-3/AR-Zellen) oder parallel transient transfiziert wurden. Weiterhin bevorzugt ist die Verwendung des TK minimal Promotors. Wenn PC-3/AR-Zellen mit einem Expressionsvektor für den Androgen-Rezeptor und einem Konstrukt aus 2 Kopien des erfindungsgemäßen HREs, dem TK minimal Promotor und dem Luciferase-Gen transfiziert werden und das Androgen R1881 (C. Bonne und J-P. Raynaud 1975, Steroids 26, 227-232) zugegeben wird, wird eine 10,5 -fache Induktion der Luciferase-Aktivität gemessen (s. Beispiel 3 und Abb. 4). Wenn PC-3/AR Zellen mit einem Expressionsvektor für den Glucokortikoid-Rezeptor und einem Konstrukt aus 2 Kopien des erfindungsgemäßen HREs, dem TK minimal Promotor und dem Luciferase-Gen transfiziert werden und das Glucocortikoid Dexamethason zugegeben wird, wird nur eine 1,9-fache Induktion gemessen. Wenn statt 2 Kopien des erfindungsgemäßen HREs 4 Kopien in demselben System eingesetzt werden, wird eine 46-fache Erhöhung der Luciferaseaktivität im Falle des R1881 und eine 1,8-fache Erhöhung mit Dexamethason gemessen. Wenn PC-3/AR-Zellen mit einem Expressionsvektor für den ProgesteronRezeptor und einem Konstrukt aus 4 Kopien des erfindungsgemäßen HREs, dem TK minimal Promotor und dem Luciferase-Gen transfiziert werden und das Progestin Levonogestrel zugegeben wird, wird eine 52,2-fache Induktion gemessen (siehe Beispiel 3 und Abb. 5). Ein Progestin ist ein synthetisches Progesteron-Analoga. Die Verwendung der Testsysteme ist nicht auf die in den Beispielen beschriebenen Testsubstanzen beschränkt, vielmehr können diese Testsyteme zum Screenen von großen Substanzbibliotheken eingesetzt werden.

Gegenstand der Erfindung ist ferner ein Verfahren zur Detektion von Testsubstanzen unter Verwendung der erfindungsgemäßen Testsysteme.

Die Erfindung liefert ferner ein Verfahren zur Bereitstellung pharmazeutisch wirksamer Substanzen wobei
a. die zu testende Substanzen mit einem erfindungsgemäßen Testsystem in Kontakt gebracht werden,
b. die Wirkung der Substanzen auf das Testsystem im Vergleich zu Kontrollen gemessen wird und
c. eine Substanz, die in Schritt b. eine Modulation der Expression des heterologen Polypeptids zeigt, identifiziert wird.

Die Erfindung betrifft ferner ein Verfahren zur Bereitstellung eines pharmazeutischen Mittels, wobei
a. die zu testende Substanzen mit einem erfindungsgemäßen Testsystem in Kontakt gebracht werden,
b. die Wirkung der Substanzen auf das Testsystem gegebenenfalls im Vergleich zu Kontrollen gemessen wird,
c. eine Substanz, die in Schritt b. eine Modulation der Expression des heterologen Polypeptids zeigt, identifiziert wird
d. und die in Schritt c. identifizierte Substanz mit der in der Pharmazie üblichen Formulierungsstoffen gemischt wird.

Die Erfindung liefert ferner ein Verfahren zur Bereitstellung eines pharmazeutischen Mittels, wobei
a. Substanzen mit einem erfindungsgemäßen Testsystem in Kontakt gebracht werden,
b. die Wirkung der Substanzen auf das Testsystem im Vergleich zu Kontrollen gemessen wird,
c. eine Substanz, die in Schritt b. eine Modulation der Expression des heterologen Polypeptids zeigt, identifiziert wird,
d. die in Schritt c. identifizierte Substanz gegebenenfalls optimiert wird und
e. diese gegebenenfalls optimierte Substanz mit in der Pharmazie üblichen Formulierungsstoffen gemischt wird.

Bevorzugt sind Substanzen, die in den erfindungsgemäßen Testsystemen die Reportergen-Aktivität mindestens 10-fach steigern oder inhibieren. Eine Substanz, die durch ein erfindungsgemäßes Verfahren identifiziert wird, kann gegebenenfalls bezüglich metabolischer Stabilität, Aktivität in einem erfindungsgemäßen Testsystem und/oder Bioverfügbarkeit optimiert werden. Dafür können in der Chemie gängige Methoden verwendet werden.
Die bevorzugten Zubereitungen bestehen in einer Darreichungsform, die zur oralen, enteralen oder parenteralen Applikation geeignet ist. Solche Darreichungsformen sind beispielsweise Tabletten, Filmtabletten, Dragees, Pillen, Kapseln, Pulver oder Depotformen sowie Suppositorien. Entsprechende Tabletten können beispielsweise durch Mischen des Wirkstoffs mit bekannten Hilfsstoffen, beispielweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmittel wie Carboxypolymethylen, Carboxymethylcellulose, Celluloseacetatphthalat oder Polyvinylacetat, erhalten werden. Die Tabletten können auch aus mehreren Schichten bestehen.
Entsprechend können Dragees durch Überziehen von analog den Tabletten hergestellten Kernen mit üblicherweise in Drageeüberzügen verwendeten Mitteln, beispielweise Polyvinylpyrrolidon oder Schellack, Gummiarabicum, Talk, Titanoxid oder Zucker, hergestellt werden. Dabei kann auch die Drageehülle aus mehreren Schichten bestehen, wobei die oben bei den Tabletten erwähnten Hilfsstoffe verwendet werden können. Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Die erfindungsgemäßen Substanzen können auch in geeigneten Lösungen wie beispielsweise physiologischer Kochsalzlösung, als Infusions- oder Injektionslösung zur Anwendung kommen. Für die parenterale Applikation sind insbesondere ölige Lösungen, wie zum Beispiel Lösungen in Sesamöl, Rizinusöl und Baumwollsamenöl, geeignet. Zur Erhöhung der Löslichkeit können Lösungsvermittler, wie zum Beispiel Benzylabenzoat oder Benzylalkohol, zugesetzt werden. Es ist auch möglich, die über das erfindungsgemäße Verfahren erhältlichen und erhaltenen Substanzen in ein Transdermales System einzuarbeiten und sie damit transdermal zu applizieren.

Mit dem erfindungsgemäßen Verfahren zur Detektion von Testsubstanzen können auch solche Substanzen bereitgestellt werden, die eine selektive Bindung eines Polypeptids, vorzugsweise des Androgenrezeptors oder des Progesteronrezeptors an das erfindungsgemäße HRE gegenüber der bekannten Konsensus-Sequenz vermitteln.

Das erfindungsgemäße pharmazeutischen Mittel kann zur Herstellung eines Medikaments für die Behandlung von Androgen-oder Progesteron-abhängigen Erkrankungen verwendet werden. Solche Erkrankungen können z.B. Prostata-Karzinom oder Hoden-Tumor sein.

Das erfindungsgemäße pharmazeutischen Mittel kann zur Herstellung eines Medikaments für die Fertilitätskontrolle verwendet werden. Für die männliche Fertilitätskontrolle kann z.B. die Expression von Genen, die für die Bildung reifer Spermien notwendig sind, inhibiert werden.

### Beschreibung der Abbildungen

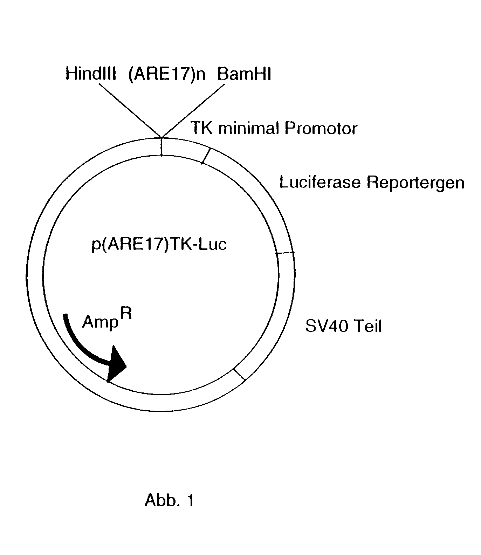

Abb. 1 zeigt die Karte des Reporterplasmids, das das erfindungsgemäße HRE genannt ARE17 mit der Sequenz entsprechend Seq ID No. 1 als 1-4 Kopien enthält.

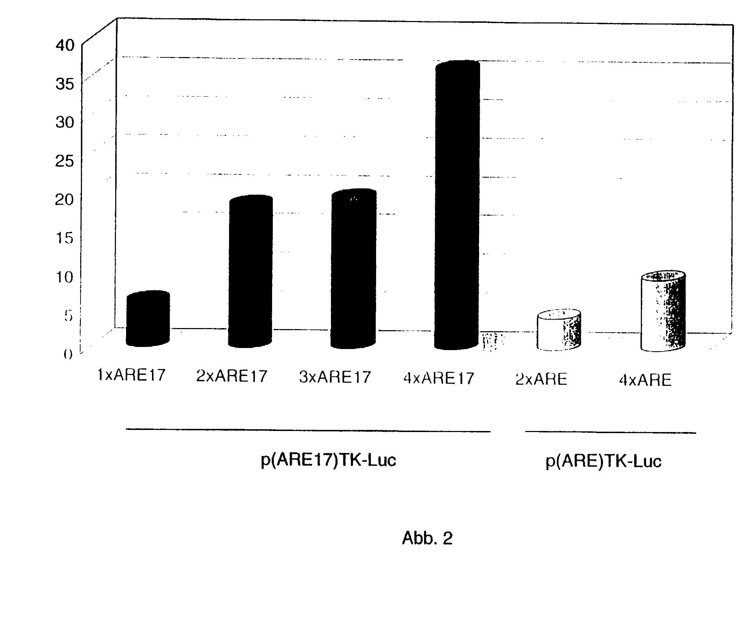

Abb. 2 zeigt die Stimulierung des erfindungsgemäßen ARE17 durch ein Androgen im Vergleich zu einem Konsensus HRE (hier ARE genannt). Die angegebene Kopiezahl des Elements wurde in den Reportervektor eingefügt. Der wurde in PC-3/AR-Zellen transfiziert und die Luciferase-Aktivität nach R1881- Behandlung gemessen. Die y- Skala zeigt an, wievielfach die Stimulation im Vergleich zu nicht mit R1881 behandelten Zellen war.

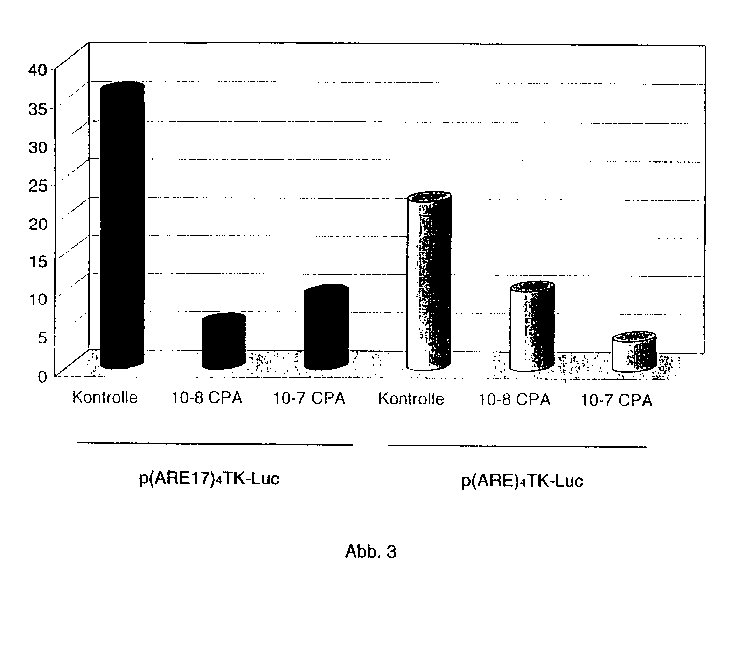

Abb. 3 zeigt die Hemmung des erfindungsgemäßen ARE17s im Vergleich zu einem Konsensus-HRE (hier ARE genannt) durch ein Antiandrogen. Vier Kopien des Elements wurden in den Reportervektor eingfügt. Der wurde in PC-3/AR-Zellen transfiziert und die Luciferase-Aktivität nach R1881- und Cyproteronacetat-Behandlung gemessen. Die y-Skala zeigt an, wievielfach die Stimulation im Vergleich zu nicht mit Cyproteronacetat behandelten Zellen war. Die Konzentrationen des Cyproteronacetats (CPA) sind in mol/l angegeben.

Abb. 4 zeigt die Stimulierung des erfindungsgemäßen ARE17s mit einem Androgen bzw. einem Glucokortikoid im Vergleich zu einem Konsensus-HRE (hier ARE genannt). Eine bis vier Kopien der Elements wurden in den Reportervektor eingefügt. Der wurde in PC-3/AR-Zellen transfiziert und die Luciferase-Aktivität nach R1881- bzw. Dexamethason-Behandlung gemessen. Die y-Skala zeigt an, wievielfach die Stimulation im Vergleich zu nicht mit Androgen oder Glucocortikoid behandelten Zellen war. Die Konzentrationen von R 1881 und von Dexamethason (Dex) sind in mol/l angegeben.

Abb. 5 zeigt die Stimulierung des erfindungsgemäßen ARE17s mit einem Androgen bzw. einem Progestin im Vergleich zu einem Konsensus-HRE. Zwei bis vier Kopien der Elements wurden in den Reportervektor eingefügt. Der wurde in PC-3/AR-Zellen transfiziert und die Luciferase-Aktivität nach R1881- bzw. Levonogestrel-Behandlung gemessen. Die y-Skala zeigt an, wievielfach die Stimulation im Vergleich zu nicht mit Androgen oder Progestin behandelten Zellen war. Die Konzentrationen von R 1881 und Levonogestrel (Lev) sind in mol/l angegeben.

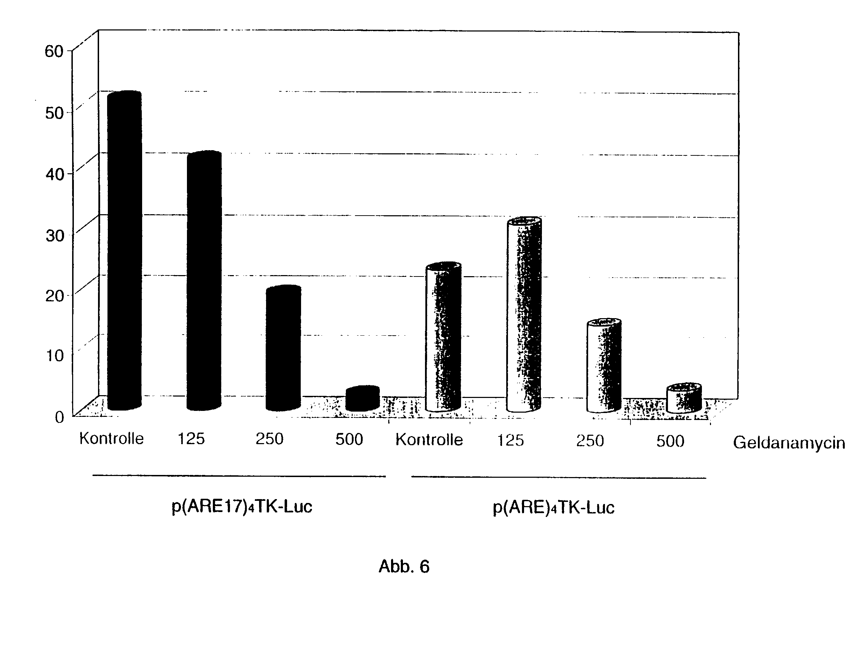

Abb. 6 zeigt die Hemmung der Androgen-Stimulierung des erfindungsgemäßen ARE17s durch Geldanamycin im Vergleich zu einem Konsensus-HRE. Vier Kopien der Elements wurden in den Reportervektor eingefügt. Der wurde in PC-3/AR-Zellen transfiziert und die Luciferase-Aktivität nach R1881- und Geldanamycin-Behandlung gemessen. Die Geldanamycin-Konzentration ist in ng/ml angegeben. In der Kontrolle wurde kein Geldanamycin eingesetzt. Die y-Skala zeigt an, wievielfach die Stimulation im Vergleich zu nicht mit Androgen behandelten Zellen war.

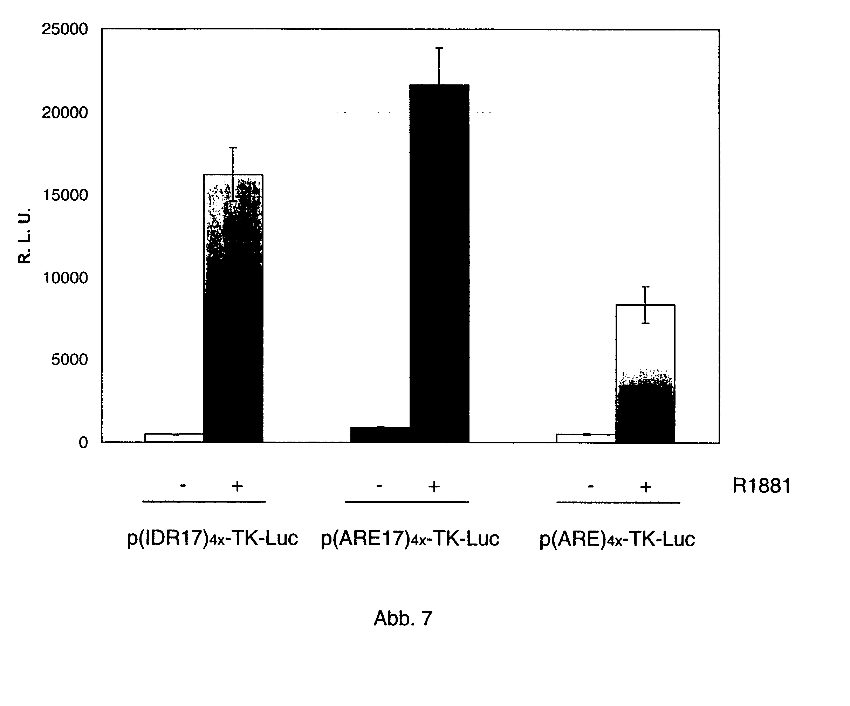

Abb.7 zeigt die Stimulation des IDR17-HREs, des erfindungsgemäßen ARE17 und des Konsensus-HREs (ARE) durch das Androgen R1881. 4x bezeichnet die Kopienzahl des Elements, das in den Reportervektor eingefügt wurde. Der wurde in PC-3/AR-Zellen transfiziert und die Luciferase-Aktivität nach R1881- Behandlung gemessen. Die y- Skala zeigt die Luciferase-Aktivität in "relative light units (R.L.U.)" an.

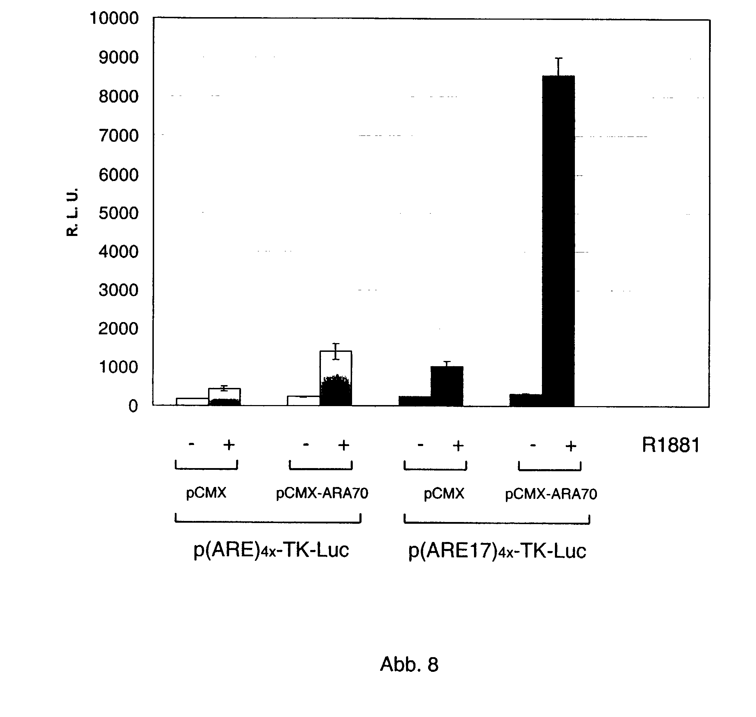

Abb. 8 zeigt den Effekt der Kotransfektion mit dem Expressionskonstrukt ohne (pCMX) und mit dem Koaktivator ARA70-Insert (pCMX-ARA70) auf die Androgenstimulierung des Konsensus-HRE (ARE) und des erfindungsgemäßen ARE17. 4x bezeichnet die Kopienzahl des Elements, das in den Reportervektor eingefügt wurde. Die y- Skala zeigt die Luciferase-Aktivität in "relative light units (R.L.U.)" an.

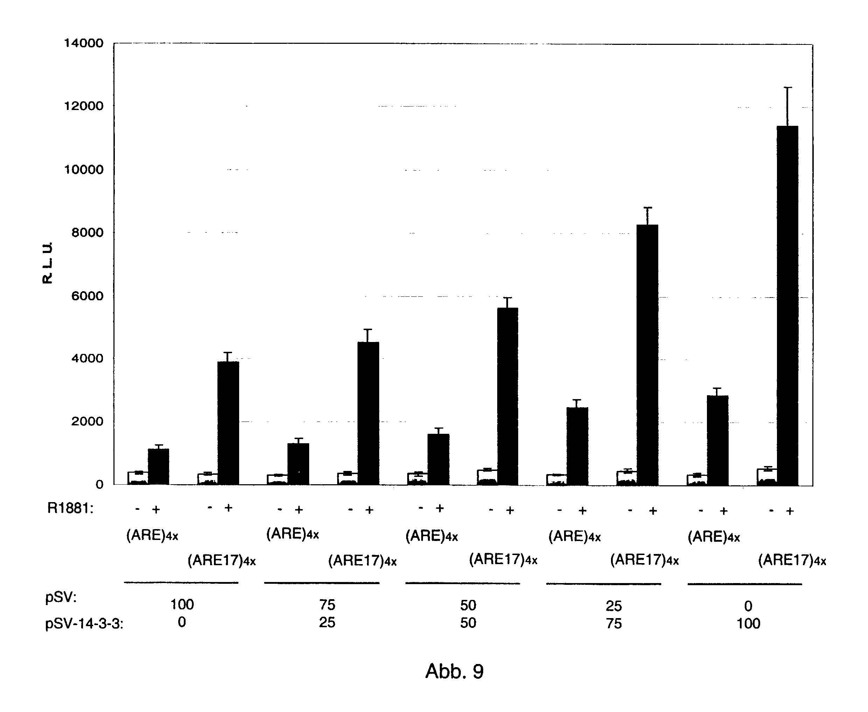

Abb. 9 zeigt den Effekt der Kotransfektion mit dem Expressionskonstrukt ohne (pSV) und mit dem 14-3-3η-Insert (pSV-14-3-3) auf die Androgenstimulierung des Konsensus-HRE (ARE) und des erfindungsgemäßen ARE17. 4x bezeichnet die Kopienzahl des Elements, das in den Reportervektor eingefügt wurde. Angegeben sind die jeweils transfizierten Mengen an pSV und pSV-14-3-3 in ng Plasmid. Die transfizierte Gesamtmenge dieser beiden Plasmide wurde auf 100 ng konstant gehalten. Die y- Skala zeigt die Luciferase-Aktivität in "relative light units (R.L.U.)" an.

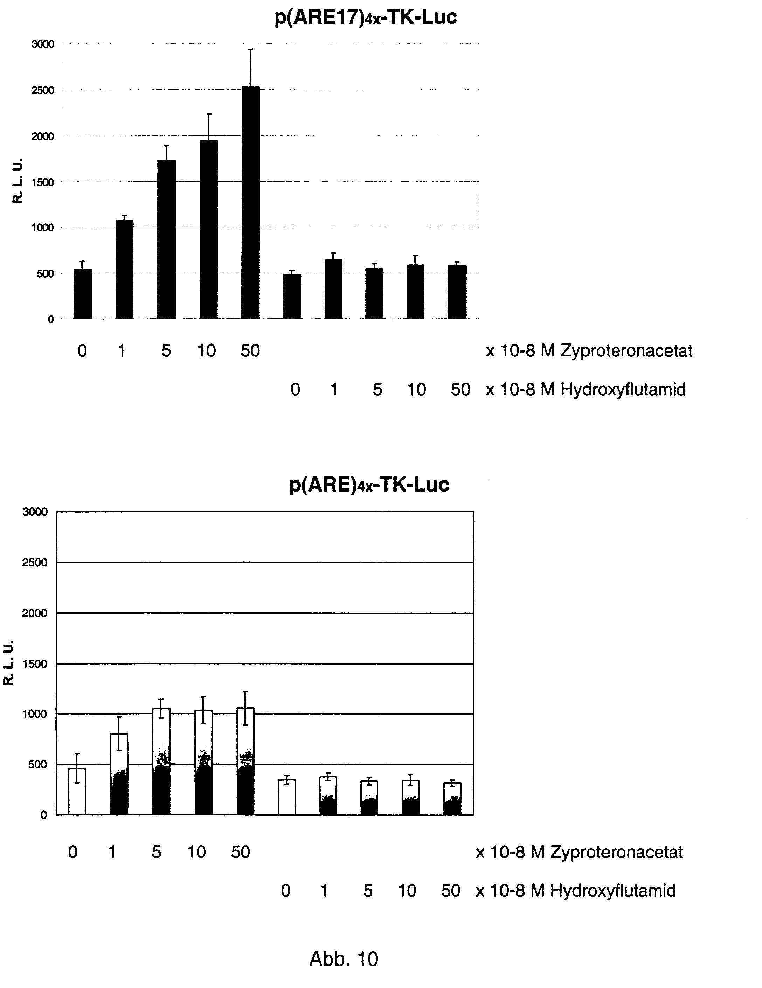

Abb. 10 zeigt die Stimulierung des erfindungsgemäßen ARE17 und der Konsensus-AREs durch Cyproteronacetat und Hydroxyflutamid in den angegebenen Konzentrationen. 4x bezeichnet die Kopienzahl des Elements, das in den Reportervektor eingefügt wurde. Die y-Skala zeigt die Luciferase-Aktivität in "relative light units (R.L.U.)" an.

### Beispiele

Die in den Beispielen verwendeten molekularbiologischen Methoden wie z.B. Polymerase-Kettenreaktion (PCR), Herstellung von cDNA, Klonierung von DNA, Sequenzierung von DNA, Transfektion, Bestimmung der Luciferase-Aktivität, wurden wie in bekannten Lehrbüchern wie zum Beispiel in Molecular Cloning, A Laboratory Manual (Sambrook et al., 1989, Cold Spring Harbor Laboratory Press) beschrieben, durchgeführt.

### Beispiel 1: Androgenabhängigkeit der Pem-Genexpression im Nebenhoden

Mäuse wurden durch tägliche Behandlung mit einem GnRH-Antagonist chemisch kastriert (B. Haendler et al. 1997, Eur. J. Biochem. 250, 440-446). Durch Kastration wird die Androgen-Biosynthese unterbunden. Nach 1 bzw. 2 Wochen wurde der Nebenhoden entfernt und RNA daraus isoliert. Diese wurde anschließend in cDNA umgewandelt und für PCR eingesetzt, die mit Hilfe von Pem-spezifischen Oligonukleotid-Primern durchgeführt wurde. Nach Trennung auf einem Agarose-Gel konnten die Mengen an Pem Amplifikationsprodukt verglichen werden. Die Ergebnisse zeigten, daß die Pem-Expression nach Kastration sehr stark reduziert war. Nur ca. 1 % der Kontrollmengen wurde gemessen. Dies deutet daraufhin, daß sehr starke Androgen Response Elemente im Pem-Gen vorhanden sein müssen.

### Beispiel 2: Identifizierung des erfindungsgemäßen HREs

Eine Analyse der publizierten Pem-Promotorregion aus der Ratte zeigt keine Sequenz die dem Konsensus-HRE nah verwandt ist. Es gibt lediglich kürzere Bereiche, die dem halben Element TGTTCT ähnlich sind und von denen nicht bekannt ist, ob sie ein HRE darstellen. Einer dieser Bereiche zeigte überraschenderweise in den im Beispiel 3 beschriebenen Tests eine sehr starke Androgeninduktion. Diesen Bereich nannten wir ARE17. Seine Sequenz entspricht Seq. ID No1.

### Beispiel 3: Nachweis des erfindungsgemäßen HREs in der Maus Pem-Promotorregion

Ein 310 Basenpaare-langes Promotorfragment wurde aus genomischer DNA der Maus durch PCR amplifiziert, mit Hilfe des GenomeWalker Kits (Clontech). Dafür wurden ein Antisens Primer 5'-GTTCTTCCGAGTCTTCCTTGAC-3' (spezifisch für das Pem-Gen) und ein Sens Primer 5'-GTAATACGACTCACTATAGGGC-3' (der eine Adaptorsequenz am Ende der genomischen DNA-Fragmenten erkennt) eingesetzt. Nach Gelreinigung wurde das amplifizierte Fragment in ein Plasmid kloniert und sequenziert. Die Sequenz ist in Seq. ID No2 dargestellt. Die Translationsinitiationsstelle ist an Position 313-315 (ATG). Sie ist mit der Pem-Promotorregion (Position 1-312) aus der Ratte zu 88,5 % identisch. Sie enthält das ARE17 mit der in Seq. ID No 1 angegebenen Sequenz an gleicher Stelle (Position 228-244) wie die Promotorregion aus der Ratte.

### Beispiel 4: Testsysteme

Es wurden Testsysteme verglichen, die entweder ein erfindungsgemäßes HRE entsprechend Seq. ID No 1 (ARE17) oder das Konsensus-HRE mit der Sequenz 5'-GGTACATCTTGTTCA-3' (ARE) enthielten. Alle anderen Komponenten waren jeweils gleich. Kopien des ARE17 wurden stromaufwärts des TK Minimalpromotors und des Luciferasegens in den pGL3-Basic Vektor eingefügt (Abb. 1). PC-3/AR-Zellen, die bereits stabil den Androgenrezeptor exprimieren, wurden ausgesät in RPMI1640/10% cFCS/L-Glutamin während 18-24 Stunden und dann fünf Stunden-lang transfiziert mit 100 ng Reporterplasmid und dem FuGene Transfektionsreagenz (Boehringer Mannheim). Danach wurde das Medium durch RPMI1640/5% cFCS/L-Glutamin ersetzt und 42 Stunden später die Aktivität des Reporter-Gens gemessen. Eine sehr starke Induktion der Luciferase-Aktivität in Gegenwart des Androgens R1881 (10⁻⁷ M) wurde gemessen (Abb. 2). Die Induktion stieg mit der Anzahl an ARE17-Kopien im Reportervektor und war stärker als die die man mit dem Konsensus-HRE beobachtet.

Die Induktion von ARE17 und von dem Konsensus-HRE ließ sich durch das Antiandrogen Cyproteronacetat (10⁻⁷ M und 10⁻⁸ M) hemmen (Abb. 3).

Wurden diese Tests in Abwesenheit eines Androgens durchgeführt, zeigte Cyproteronacetat eine dosis-abhängige Stimulation von ARE17 während es nur einen sehr geringen oder keinen Effekt auf das Konsensus-HRE hatte (Abb.10). Das Antiandrogen Hydroxyflutamid dagegen stimulierte weder ARE17 noch das Konsensus-HRE.

In weiteren Versuchen wurde die Stimulation des erfindungsgemäßen HREs (ARE17), des Konsensus-HREs mit der Sequenz 5'-GGTACATCTTGTTCA-3' oder das IDR17-Element mit der Sequenz 5'-GGAACGGAACATGTTCT-3'(Zhou et al. 1997, J. Biol. Chem. 272, 8227-8235) verglichen. Alle anderen Komponenten waren jeweils gleich. Vier Kopien der Elementen wurden stromaufwärts des TK Minimalpromotors und des Luciferasegens in den pGL3-Basic Vektor eingefügt (Abb. 1 ) und in PC-3/AR-Zellen, die bereits stabil den Androgenrezeptor exprimieren, transfiziert, wie oben beschrieben. Die Stimulierung erfolgte mit dem synthetischen Androgen R1881 (10⁻⁹ M oder 10⁻⁷ M Endkonzentration). Es wurde keine bedeutender Unterschied zwischen beiden Mengen gesehen.
Ein Vergleich zwischen dem erfindungsgemäßen HRE (ARE17), dem Konsensus-HRE (ARE) und dem IDR17-Element zeigte die stärkste Induktion der Luciferase-Aktivität in Gegenwart des Androgens R1881 für das erfindungsgemäße HRE (ARE17) während das Konsensus-HRE (ARE) die schwächste Stimulierung zeigte (Abb. 7).

Die zusätzliche Transfektion mit dem pCMX-ARA70 Expressionskonstrukt (100 ng), das für den Androgenrezeptor-selektiven Koaktivator ARA70 kodiert (Yeh and Chang 1996, Proc. Natl. Acad. Sci. 93, 5517-5521), zeigte eine Stimulierung durch R1881 gegenüber der Kontrolle (100 ng Expressionsvektor pCMX ohne Insert) die viel ausgeprägter war für das erfindungsgemäße HRE (ARE17) als für das Konsensus-HRE (ARE; Abb. 8). Die Kotransfektion mit dem pSV-14-3-3η Expressionskonstrukt, das für das Protein 14-3-3η kodiert (Muratake et al. 1995, Mol. Neurobiol. 11, 223-230), bewirkte eine Dosis-abhängige Stimulierung der Androgen-Antwort, die für das erfindungsgemäße HRE (ARE17) ausgeprägter war als für das Konsensus-HRE (ARE; Abb. 9).

Ein großer Unterschied wurde gesehen, wenn man die ARE17- und Konsensus-HRE-Konstrukte nach Kotransfektion der PC-3/AR-Zellen mit einem Expressionsvektor für den Glucokorticoidrezeptor mit Dexamethason (10⁻⁸ M) behandelte. Hier wurde praktisch keine Stimulierung für das ARE17 gesehen, dafür ein mit der R1881-Stimulation vergleichbarer Effekt für das Konsensus-HRE (Abb. 4). Weiterhin wurde festgestellt, daß das ARE17 sehr stark durch das Progestin Levonogestrel (10⁻⁶ M), nach Kotransfektion mit einem Expressionsvektor für den Progesteronrezeptor, stimulierbar ist (Abb. 5). Behandlung durch Geldanamycin, eine Substanz die die Aktion von Steroidrezeptoren hemmt, zeigte, daß eine niedrige Konzentration (125 ng/ml) die Androgenstimulation des ARE17 hemmt, aber die des Konsensus-HRE erhöht (Abb. 6).

### Anlage zur Beschreibung

## Patentansprüche

1. HRE (Hormon Response Element, Hormonrezeptorbindungstelle), das den Androgen- und den Progesteronrezeptor bindet, dadurch gekennzeichnet, daß das HRE
a. eine DNA-Sequenz der allgemeinen Formel (N)ₓTCTCATTCTGTTCC, wobei N unabhängig voneinander und in beliebiger Kombination A, T, C oder G ist und x gleich 0-3 ist,
b. eine Variation der unter a. genannten Sequenz oder
c. eine DNA-Sequenz, die komplementär zu der unter a. oder b. genannten DNA-Sequenz ist,
umfaßt.

2. HRE nach Anspruch 1, wobei x = 0 ist und die DNA-Sequenz Seq. ID No.3 entspricht.

3. HRE nach Anspruch 1, wobei x = 3 ist.

4. HRE nach Anspruch 3, wobei N = AGA ist und die DNA-Sequenz Seq. ID No 1 entspricht.

5. Verwendung einer HRE-DNA-Sequenz nach einem der Ansprüche 1-4 zur Detektion von Genen, die durch Androgen und/oder Progesteron reguliert werden.

6. Verwendung einer HRE-DNA- Sequenz nach einem der Ansprüche 1-4 oder einer durch eine HRE-DNA- Sequenz nach einem der Ansprüche 1-4 regulierten Sequenz zur Detektion einer Veränderung der Expression von Androgen oder/und Progesteron regulierten Genen in erkrankten Zellen gegenüber normalen Zellen.

7. Verwendung einer HRE-DNA-Sequenz nach einem der Ansprüche 1-4 zur Detektion von Mutationen in Expressions-Regulationselementen, insbesondere HRE's, die durch Androgen und/oder Progesteron reguliert werden.

8. Verwendung nach Anspruch 6 oder 7 zur Detektion von Veränderungen in Tumorzellen.

9. Vektor umfassend ein HRE nach einem der Ansprüche 1-4 in einer oder mehreren Kopien, in operativer Verknüpfung mit dem zu exprimierenden Gen.

10. Vektor nach Anspruch 9 dadurch gekennzeichnet, daß die operative Verknüpfung der TK minimal Promotor ist.

11. Zellen transfiziert mit einer Sequenz nach einem der Ansprüche 1-4 oder mit einem Vektor nach Anspruch 9 oder 10.

12. Zellen nach Anspruch 11 dadurch gekennzeichnet, daß die Zellen aus der Gruppe bestehend aus PC-3-Zellen, LNCaP-Zellen, CV-1-Zellen, HeLa-Zellen und Dunning-Zellen ausgewählt sind.

13. Verwendung einer Zelle nach Anspruch 11 oder 12 zur Expression des mit der HRE-Sequenz operativ verknüpften Gens.

14. Verwendung nach Anspruch 13, wobei die Zelle ein mit dem HRE-Element bindefähiges Polypeptid, welches eine zusätzliche Ligandenbindedomäne aufweist, enthält.

15. Testsystem für hormonrezeptorbindungsfähige Substanzen umfassend
a. eine DNA-Sequenz nach einem der Ansprüche 1-4 in operativer Verknüpfung mit einem zu exprimierenden oder zu transkribierenden heterologen Gen und
b. ein Polypeptid, das mit der DNA nach einem der Ansprüche 1-4 bindefähig ist.

16. Testsystem nach Anspruch 15 zur Detektion von Testsubstanzen mit androgener Aktivität, wobei
a. in Zellen nach Anspruch 11 oder 12 ein Reportergen exprimiert wird und
b. diese Zellen, wenn sie keinen Androgenrezeptor enthalten, zusätzlich mit einem Expressionsvektor für den Androgenrezeptor transfiziert werden,
c. die Zellen in Gegenwart oder Abwesenheit der Testsubstanzen kultiviert werden und
d. die Veränderung der Expression des Reportergens gemessen wird.

17. Testsystem nach Anspruch 15 zur Detektion von Testsubstanzen mit antiandrogener Aktivität, wobei
a. in Zellen nach Anspruch 11 oder 12 ein Reportergen exprimiert wird und
b. diese Zellen, wenn sie keinen Androgenrezeptor enthalten, zusätzlich mit einem Expressionsvektor für den Androgenrezeptor transfiziert werden,
c. die Zellen in Gegenwart oder Abwesenheit der Testsubstanzen bei gleichzeitiger Anwesenheit eines Androgens kultiviert werden und
d. die Veränderung der Expression des Reportergens gemessen wird.

18. Testsystem nach Anspruch 15 zur Detektion von Testsubstanzen mit Progestin-Aktivität, dadurch gekennzeichnet, daß
a. in Zellen nach Anspruch 11 oder 12 ein Reportergen exprimiert wird und
b. diese Zellen, wenn sie keinen Progesteronrezeptor enthalten, zusätzlich mit einem Expressionsvektor für den Progesteronrezeptor transfiziert werden,
c. die Zellen in Gegenwart oder Abwesenheit der Testsubstanzen kultiviert werden und
d. die Veränderung der Expression des Reportergens gemessen wird.

19. Testsystem nach Anspruch 15 zur Detektion von Testsubstanzen mit anti-Progestin Aktivität, dadurch gekennzeichnet, daß
a. in Zellen nach Anspruch 11 oder 12 ein Reportergen exprimiert wird und
b. diese Zellen, wenn sie keinen Progesteronrezeptor enthalten, zusätzlich mit einem Expressionsvektor für den Progesteronrezeptor transfiziert werden,
c. die Zellen in Gegenwart oder Abwesenheit der Testsubstanzen bei gleichzeitiger Anwesenheit eines Progestins kultiviert werden und
d. die Veränderung der Expression des Reportergens gemessen wird.

20. Testsystem nach einer der Ansprüche 15-19 dadurch gekennzeichnet, daß das Reportergen aus der Gruppe bestehend aus Luciferasegen, Chloramphenicolacetyltransferasegen, Urokinasegen, Green Fluorescence Protein-Gen und β-Galaktosidase ausgewählt ist.

21. Verfahren zur Detektion von Testsubstanzen unter Verwendung eines der Testsysteme gemäß den Ansprüchen 15-20.

22. Verfahren zur Bereitstellung pharmazeutisch wirksamer Substanzen, wobei
a. Substanzen mit einem Testsystem nach einem der Ansprüche 15-20 in Kontakt gebracht werden,
b. die Wirkung der Substanzen auf das Testsystem im Vergleich zu Kontrollen gemessen wird und
c. eine Substanz, die in Schritt b. eine Modulation der Expression des heterologen Polypeptids zeigt, identifiziert wird.

23. Verfahren zur Bereitstellung eines pharmazeutischen Mittels, wobei
a. Substanzen mit einem Testsystem nach einem der Ansprüche 15-20 in Kontakt gebracht werden,
b. die Wirkung der Substanzen auf das Testsystem im Vergleich zu Kontrollen gemessen wird,
c. eine Substanz, die in Schritt b. eine Modulation der Expression des heterologen Polypeptids zeigt, identifiziert wird
d. und die in Schritt c. identifizierte Substanz mit in der Pharmazie üblichen Formulierungsstoffen gemischt wird.

24. Verfahren zur Bereitstellung eines pharmazeutischen Mittels, wobei
a. Substanzen mit einem Testsystem nach einem der Ansprüche 15-20 in Kontakt gebracht werden,
b. die Wirkung der Substanzen auf das Testsystem im Vergleich zu Kontrollen gemessen wird,
c. eine Substanz, die in Schritt b. eine Modulation der Expression des heterologen Polypeptids zeigt, identifiziert wird,
d. die in Schritt c. identifizierte Substanz gegebenenfalls optimiert wird und
e. diese gegebenenfalls optimierte Substanz mit in der Pharmazie üblichen Formulierungsstoffen gemischt wird.

25. Verwendung einer nach Anspruch 22 bereitgestellten Substanz oder eines nach Anspruch 23 oder 24 bereitgestellten pharmazeutischen Mittels zur Herstellung eines Medikaments für die Behandlung von Androgen-oder Progesteron-abhängigen Erkrankungen.

26. Verwendung einer nach Anspruch 22 bereitgestellten Substanz oder eines nach Anspruch 23 oder 24 bereitgestellten pharmazeutischen Mittels zur Herstellung eines Medikaments für die Fertilitätskontrolle.


SEQUENZPROTOKOLL

<110> Schering Aktiengesellschaft

<120> Androgen- und Progesteron-Response Element

<130> 51619AEPM1XXOO-P

<140>
<141>

<150> DE 198 55 013.8-44
<151> 1998-11-20

<160> 3

<170> PatentIn Ver. 2.1

<210> 1
<211> 17
<212> DNA
<213> Rattus norvegicus

<400> 1
agatctcatt ctgttcc 17

<210> 2
<211> 385
<212> DNA
<213> Mus musculus

<400> 2
tgagctgtaa ctgggcaccc taagttctgc acacccacat gcccatgaac tgtgtccatc 60
ttgcaagcac atcgtgctca ttacatcccc aaactgctat cacttgtgta ccccaaaggc 120
tcggcccaca ggaacgtcct gtgagcaaat cacaaagacc agcttagggc tggaaacatt 180
gtaacctgaa gtaggccaga ggagatccct gccaggttga gcatcacaga tctcattctg 240
ttcccgggga caccaggggc ccaagctcag aatctgccga agcataactt catcattgat 300
cctattcagg gtatggaagc tgagggttcc agccgcaagg tcaccaggct actccgcctg 360
ggagtcaagg aagactcgga agaac 385

<210> 3
<211> 14
<212> DNA
<213> Mus musculus

<400> 3
tctcattctg ttcc 14